# EUROPEAN PATENT SPECIFICATION

(11) **EP 0 376 215 B1**
(45) Date of publication and mention of the grant of the patent: **24.05.1995**
(21) Application number: 89123826.3
(22) Date of filing: 22.12.1989
(51) Int. Cl.: C07D 233/64, C07C 277/08, C07C 59/185, C07C 229/26, C07C 59/84

(54) **alpha-Keto acid/amino acid salt compounds and a process for their production**
Alpha-Ketosäure/Aminosäure-Salz-Verbindungen und Verfahren zu ihrer Herstellung
Sels d'alpha-cétoacides avec aminoacides et leur procédé de préparation

(30) Priority: 27.12.1988 JP 330688/88
(43) Date of publication of application: 04.07.1990
(73) Proprietor: AJINOMOTO CO., INC., Tokyo 104 (JP)
(72) Inventor: Tanabe, Toshiya Central Research Lab., Kawasaki-shi Kanagawa-ken (JP); Kishimoto, Shin-ichi Central Research Lab., Kawasaki-shi Kanagawa-ken (JP)
(74) Representative: Strehl Schübel-Hopf Groening & Partner

(56) References cited:
- FR-A- 1 432 756
- FR-A- 2 222 370
- FR-A- 2 454 434
- GB-A- 2 017 094

## Description

The present invention relates to a novel α-keto acid/amino acid salt compound and a process for its production.

Amino acid salts of α-keto acids are effective for the treatment of patients with renal disorders caused by uremia, etc. and with hepatic disorders caused by hyperammoniemia, etc. With respect to the crystals, anhydrous crystals or those containing a very small amount of water have been described (Japanese Patent Publication Nos. 58-38421 and 60-24094).

Amino acid salts of α-keto acids have a very high solubility in water (50 to 70 wt%). This property is advantageous since it is unnecessary to give the patients to be treated excessive amounts of water in treatment. These salts cause, however, difficulties in crystallization, resulting in serious obstacles in the process of their production.

For isolating crystals of the amino acid salts of an α-keto acid, methods, such as freeze drying, crystallization with solvent and the like are hitherto known. Regarding the production in an industrial scale, solvent crystallization is practical. However, when solvent crystallization is performed, it is extremely difficult to remove the solvent. In particular, since the amino acid salts of an α-keto acid are used as drugs for treating patients with renal disorders or hepatic disorders, a strict standard is applied for the residual solvent so that a very long time period is required for drying. Furthermore, the amino acid salts of an α-keto acid are sensitive to heat and liable to be colored so that drying should be performed at a very low temperature in vacuum. Even under such conditions, the dissolution state is deteriorated depending upon the kind of the salts.

The problem to be solved by the invention is to develop a method which allows the production of amino acid salts of α-keto acids effective for treating renal or hepatic disorders in a smaller number of process steps in a short period of time, without causing decomposition or denaturation at high temperatures.

As a result of extensive investigations to solve the foregoing problems, the present inventors have found that alcohol or ketone adducts of α-keto acid/amino acid salts obtained by, e.g., solvent crystallization can solve the problems.

The present invention provides an α-keto acid/amino acid-salt represented by the following formula :

AN·xS

wherein A represents an amino acid, N represents an α-keto acid, S represents a lower alcohol or a ketone; and x represents 0.1 to 3.

These compounds are solvated compounds containing a solvent in their crystals.

The crystals of the solvated compounds of the amino acid salts of an α-keto acid are transformed by the process described in claim 5 under conditions of an absolute humidity of 0.003 to 17.0 kg-H₂O/kg-dry air, whereby desolvated α-keto acid/amino acid salts compounds can be produced.

When the concentration of the solvent is reduced upon the crystallization of the α-keto acid/amino acid salt compound, it is possible to isolate non-solvated crystals. However, the amino acid salts of α-keto acid have a very high solubility in water and, if water is the solvent, the solubility is not lowered until the solvent concentration becomes considerably low so that the yield is markedly reduced in crystallization in a low solvent concentration. In contrast thereto, according to the process of the present invention, crystallization can be effected even in a high solvent concentration and it is possible to increase the yield.

The amino acid salts of α-keto acids which are used in the present invention are not particularly limited. Examples of α-keto acids are α-keto-isocaproic acid, α-keto isovaleric acid, α-keto-β-methylvaleric acid, pyruvic acid and phenyl-pyruvic acid, etc. The amino acids are basic or neutral amino acids capable of forming salts with α-keto acids, for example, histidine, lysine, ornithine, arginine, etc. Examples of the solvents are lower alcohols exemplified by methanol, ethanol, n-propanol, isopropanol, etc. or those miscible with water such as ketones, like acetone, etc.

The crystallization of the solvated α-keto acid/amino acid salts can be carried out by adding an aqueous solution of the α-keto acid/amino acid salts to the aforesaid solvent or a mixture of water with the solvent in a conventional manner and allowing the resulting mixture to cool.

That is, the α-keto acid is added to a suspension or an aqueous solution of an amino acid to prepare an aqueous solution of the α-keto acid/amino acid salt compound. If necessary, adsorbents such as activated charcoal, activated alumina, bentonite, etc. are added to the mixture for decoloration and the filtrate is concentrated. The concentrate is added to the solvent or its mixture with water. The resulting mixture is cooled for crystallization.

The concentration of the aqueous solution of an α-keto acid/amino salt compound varies depending upon kind of compound but is appropriately in a range of 50 to 75 %. In order to increase the yield of the crystallization, it is preferred to use the solvent in a high concentration.

Analysis of X ray diffraction reveals that the thus crystallized solvated α-keto acid/amino acid salt compound has a crystalline shape which is obviously different prior to and after removal of the solvent and that the solvent contained in the crystals does not merely adhere to the crystals but is incorporated within the lattice of the crystals to form a solvated product. It has also been revealed that for removal of the solvent, it is necessary to transform the crystal lattice and after transformation of the crystals, it is extremely difficult to remove the residual solvent. Further even in salts showing no crystal transformation such as lysine/α-keto-β-methyl-valeric acid (Lys/KMV), etc., it takes a long time to remove the residual solvent by conventional drying merely in vacuum.

According to the present invention, removal of the solvent can be performed in a short period of time simply by keeping the crystals in an appropriate humidity under relatively mild conditions under normal pressure. It is sufficient that the humidity be set at 0.003 to 17.0 kg-H₂O/kg-dry air, irrespective of the pressure. The temperature does not very greatly affect the crystal transformation but is desirably below 60°C since the amino acid salts of α-keto acid are sensitive to heat and tend to be readily colored.

Hereafter the present invention is described in detail by referring to the examples.

### Example 1

Water (about 30 ml) sufficient to suspend 0.1 mol of histidine (about 15.5 g) was charged and 10 g of α-keto-isocaproic acid was added thereto. The mixture was changed from a slurry state to a completely dissolved state.

To the solution was added 3 g of α-keto-isocaproic acid and the pH of the mixture was adjusted to 4.0. After 1 g of activated charcoal was added, the mixture was stirred and decolored and then filtered. The filtrate was concentrated to about 47 g. The concentrate was added to 170 ml of ethanol (ethanol/water = 9.2 [v/v]). After allowing to stand at 25°C overnight, the crystals were separated and dried at 40°C for a day under 13.3 x 10⁻³ bar (10 torr) to give 31 g of about 0.9 ethanol-solvated crystals of histidine α-keto-isocaproate (ethanol content 13 %; crystallization rate 94 %).

The crystals were allowed to stand at 40°C in relative humidity of 80 % (absolute humidity of 0.03 kg-H₂O/kg-dry air) under normal pressure. About 4 hours after, cystals having an ethanol content of 320 ppm were obtained.

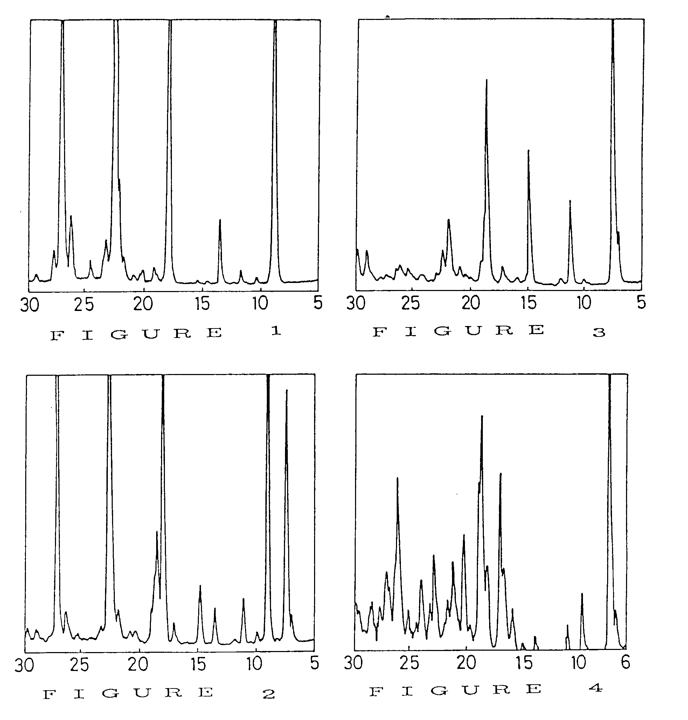

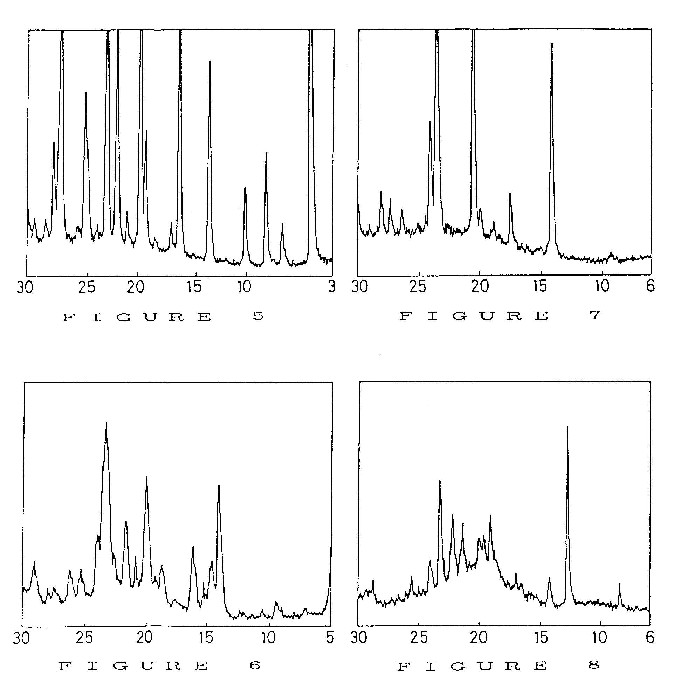

The results of the X ray diffraction pattern are shown in Figs. 1 through 3. The X ray diffraction pattern of the crystals is obviously different between before and after the removal of ethanol, indicating that ethanol is not merely adhered to but incorporated into the crystal lattice.

Crystal transformation was completed in about 2.5 hours. In this case, 0.2 % of the residual ethanol was noted but this residual ethanol could finally be removed.

The above procedure was performed by varying temperature and relative humidity for removing ethanol in the above crystals. The results are shown in Table 1.

**Table 1**

| Removal of Ethanol from L-Histidine α-Keto-isocaproate | | | | |
|---|---|---|---|---|
| Temperature [°C] | Relative Humidity [%] | Absolute Humidity [kg-H₂O/kg-dry air] | Time [hr] | Residual EtOH [ppm] |
| | 80 | 0.039 | 3 | 320 |
| | 60 | 0.029 | 3 | 350 |
| 40 | 40 | 0.020 | 24 | 90 |
| | 30 | 0.015 | 24 | 130 |
| | 25 | 0.005 | 48 | 90 |
| 25 | 60 | 0.012 | 24 | 140 |

### Comparative Example 1

A concentrated solution of histidine α-keto-isocaproate obtained in a manner similar to Example 1 was added to 22 ml of EtOH (ethanol/water = 1.19 [v/v]). After allowing to stand overnight, the crystals were separated and dried at 40°C for a day under 13.3 x 10⁻³ bar (10 torr) to give 15.1 g of ethanol-non-solvated crystals of histidine α-keto-isocaproate. The results of X ray diffraction pattern are shown in Fig. 4. The crystallization rate was 53 % in this case.

### Example 2

In 60 ml of water was dissolved 0.1 mol of L-ornithine (about 13.2 g) and about 11.6 g of α-keto-isovaleric acid was added to the solution to adjust pH to 5.9. After about 1 g of activated charcoal was added to decolor, the filtrate was concentrated to about 41 g. The concentrate was added to 140 ml of ethanol (ethanol/water = 8.6 [v/v]). After allowing to stand at 5°C overnight, the crystals were separated. The thus obtained ethanol-solvated crystals were dried at 40°C for 4 hours under 46.6 x 10⁻³ bar (35 torr) to remove the adhered ethanol. Then, the crystals were allowed to stand under conditions of relative humidity of 60 % under normal pressure. Two hours after allowing to stand under the humidity controlled conditions, the residual ethanol became 450 ppm.

### Comparative Example 2

L-Ornithine α-keto isocaproate obtained in a manner similar to Example 2 was dried in vacuum under conditions of 40°C and 46.6 x 10⁻³ bar (35 torr). The residual EtOH was 3.9 % after 12 hours passed.

### Example 3

In 60 ml of water was dissolved 0.1 mol of L-ornithine (about 13.2 g) and about 11.6 g of α-keto-isovaleric acid was added to the solution to adjust pH to 5.9. After about 1 g of activated charcoal was added to the mixture to decolor, the filtrate was concentrated to about 41 g. The concentrate was added to about 120 ml of acetone. After allowing to stand at 5°C overnight, the crystals were separated. The thus obtained acetone-solvated crystals were dried at 40°C for an hour under 46.6 x 10⁻³ bar (35 torr) to remove the adhered acetone. Then, the crystals were allowed to stand under conditions of relative humidity of 60% under normal pressure. Two hours after, the residual acetone to which moisture was given became 90 ppm. The results of X ray diffraction are shown in Figs. 5 and 6.

### Comparative Example 3

L-Ornithine α-keto isovaleric acid obtained in a manner similar to Example 3 was dried in vacuum under conditions of 40°C and 46.6 x 10⁻³ bar (35 torr). The residual acetone was 0.5 % after 5 hours passed.

### Example 4

In 60 ml of water was dissolved 0.1 mol of L-ornithine (about 13.2 g) and about 13 g of α-keto-isocaproic acid was added to the solution to adjust the pH to 5.7. After about 1 g of activated charcoal was added to decolor, the filtrate was concentrated to about 43 g. The concentrate was added to 150 ml of ethanol. After allowing to stand at 5°C overnight, the crystals were separated. The thus obtained ethanol-solvated crystals were dried at 40°C for 4 hours under 46.6 x 10⁻³ bar (35 torr) to remove the adhered ethanol. Then, the crystals were allowed to stand under conditions of relative humidity of 70 % under normal pressure. Two hours after allowing to stand under the humidity controlled conditions, the residual ethanol became 26 ppm.

### Comparative Example 4

L-Ornithine α-keto-isocaproate obtained in a manner similar to Example 4 was dried in vacuum under conditions of 40°C and 46.6 x 10⁻³ bar (35 torr). The residual ethanol was 1.1 % after 12 hours passed.

### Example 5

In 60 ml of water was dissolved 0.1 mol of L-ornithine (about 13.2 g) and about 13 g of α-keto-isocaproic acid was added to the solution to adjust pH to 5.7. After about 1 g of activated charcoal was added to decolor, the filtrate was concentrated to about 43 g. The concentrate was added to 150 ml of isopropanol. After allowing to stand at 5°C overnight, the crystals were separated. The thus obtained isopropanol-solvated crystals were dried at 40°C for 4 hours under 46.6 x 10⁻³ bar (35 torr) to remove the adhered isopropanol. Then, the crystals were allowed to stand under conditions of relative humidity of 70 % under normal pressure. Two hours after allowing to stand under the humidity controlled conditions, the residual isopropanol became 110 ppm. The results of the X ray diffraction pattern are shown in Figs. 7 and 8.

### Comparative Example 5

L-Ornithine α-keto-isocaproate obtained in a manner similar to Example 5 was dried in vacuum under conditions of 40°C and 46.6 x 10⁻³ bar (35 torr). The residual isopropanol was 2.0 % after 18 hours passed.

### Example 6

In 60 ml of water was dissolved 0.1 mol of L-lysine (about 14.6 g) and about 13 g of α-keto-β-methylvaleric acid was added to the solution to adjust the pH to 6.0. After about 1 g of activated charcoal was added to the mixture to decolor, the filtrate was concentrated to about 46 g. The concentrate was added to about 350 ml of ethanol. After allowing to stand at 5°C overnight, the crystals were separated. The thus obtained ethanol-solvated crystals were dried at 30°C for 4 hours under 46.6 x 10⁻³ bar (35 torr) to remove the adhered ethanol. Then, the crystals were allowed to stand under conditions of relative humidity of 60 %. Three hours after, the residual ethanol to which moisture was given became 250 ppm.

### Comparative Example 6

L-Lysine α-keto-β-methylvaleric acid obtained in a manner similar to Example 6 was dried in vacuum under conditions of 40°C and 46.6 x 10⁻³ bar (35 torr). It took 8 hours until the residual ethanol became a level almost equal to Example 5.

### [Effects of the Invention]

As stated above, according to the present invention, the crystals of an α-keto acid/amino acid salt compound are once crystallized as the solvated crystals and the solvent is removed from the solvated crystals, whereby the crystals of the α-keto acid/amino acid salt compound can be produced in a high yield without causing any decomposition or denaturation so that it can be expected to greatly reduce production costs.

### Brief Description of the Drawings

Fig. 1 shows an X ray diffraction pattern of EtOH-solvated His/KIC crystals obtained in Example 1 of the present invention.

Fig. 2 shows an X ray diffraction pattern an hour after the removal of EtOH from the same EtOH-solvated His/KIC crystals.

Fig. 3 shows an X ray diffraction pattern after completion of the removal of EtOH from the same EtOH-solvated His/KIC crystals.

Fig. 4 shows an X ray diffraction pattern of EtOH-non-solvated His/KIC crystals obtained in Comparative Example 1.

Figs. 5 and 6 show X ray diffraction patterns of non-solvated Orn/KIV crystals and acetone-solvated Orn/KIV crystals obtained in Example 3, respectively.

Figs. 7 and 8 show X ray diffraction patterns of non-solvated Orn/KIC crystals and isopropyl alcohol-solvated Orn/KIC crystals obtained in Example 3, respectively.

(His : histidine, Orn : ornithine, KIC : α-keto-isocaproic acid, KIV : α-keto-isovalerianic acid).

## Claims

1. An α-keto acid/amino acid-salt represented by the following formula :
AN·xS
wherein A represents an amino acid; N represents an α-keto acid; S represents a lower alcohol or a ketone; and x represents 0.1 to 3.

2. A compound according to claim 1, wherein said amino acid is selected from the group consisting of histidine, ornithine, lysine and arginine.

3. A compound according to claim 1 or 2, wherein said α-keto acid is selected from the group consisting of α-keto isocaproic acid, α-keto isovaleric acid, α-keto-β-methylvaleric acid, pyruvic and phenylpyruvic acid.

4. A compound according to any of the claims 1 to 3, wherein S is acetone.

5. A process for the production of an α-keto acid/amino acid salt which comprises transforming crystals of a compound represented by the following formula
AN·xS
wherein A represents an amino acid; N represents an α-keto acid; S represents a lower alcohol or a ketone; and x represents 0.1 to 3, under conditions of an absolute humidity of 0.003 to 17.0 kg-H₂O/kg-dry air.

## Patentansprüche

1. α-Ketosäure/Aminosäure-Salz, das durch die folgende Formel dargestellt ist:
AN·xS
worin A eine Aminosäure darstellt, N eine α-Ketosäure darstellt, S einen niederen Alkohol oder ein Keton darstellt und x 0,1 bis 3 ist.

2. Verbindung nach Anspruch 1, worin die Aminosäure aus der aus Histidin, Ornithin, Lysin und Arginin bestehenden Gruppe ausgewählt ist.

3. Verbindung nach Anspruch 1 oder 2, worin die α-Ketosäure aus der aus α-Ketoisocapronsäure, α-Ketoisovaleriansäure, α-Keto-β-methylvaleriansäure, Brenztraubensäure und Phenylbrenztraubensäure bestehenden Gruppe ausgewählt ist.

4. Verbindung nach einem der Ansprüche 1 bis 3, worin S Aceton ist.

5. Verfahren zur Herstellung eines α-Ketosäure/Aminosäure-Salzes, welches Umwandeln von Kristallen einer Verbindung unter Bedingungen einer absoluten Feuchtigkeit von 0,003 bis 17,0 kg-H₂O/kg trockene Luft umfaßt, wobei die Verbindung durch die folgende Formel dargestellt ist:
AN·xS
worin A eine Aminosäure darstellt, N eine α-Ketosäure darstellt, S einen niederen Alkohol oder ein Keton darstellt und x 0,1 bis 3 ist.

## Revendications

1. Sel d'α-cétoacide/acide aminé représenté par la formule suivante :
AN·xS
dans laquelle A représente un acide aminé, N représente un α-cétoacide, S représente un alcool inférieur ou une cétone et x est compris entre 0,1 et 3.

2. Composé selon la revendication 1, dans lequel on choisit ledit acide aminé dans le groupe constitué par l'histidine, l'ornithine, la lysine et l'arginine.

3. Composé selon la revendication 1 ou la revendication 2, dans lequel on choisit ledit α-cétoacide dans le groupe constitué par l'acide α-céto-isocaproïque, l'acide α-céto-isovalérique, l'acide α-céto-β-méthylvalérique, l'acide pyruvique et l'acide phénylpyruvique.

4. Composé selon l'une quelconque des revendications 1 à 3, dans lequel S est l'acétone.

5. Procédé de production d'un sel d'α-cétoacide/acide aminé qui comprend la transformation de cristaux d'un composé représenté par la formule suivante :
AN·xS
dans laquelle A représente un acide aminé, N représente un α-cétoacide, S représente un alcool inférieur ou une cétone et x est compris entre 0,1 et 3, dans des conditions d'une humidité absolue comprise entre 0,003 et 17,0 kg d'H₂O/kg d'air sec.
